# DEMANDE DE BREVET EUROPEEN

(11) **EP 1 254 649 A1**
(43) Date de publication de la demande: **06.11.2002**
(21) Numéro de dépôt: 02291000.4
(22) Date de dépôt: 19.04.2002
(51) Int. Cl.: A61K 7/027, A61K 7/42

(54) **Composition cosmétique comprenant un ester et un agent photoprotecteur**

(30) Priorité: 04.05.2001 FR 0106048
(71) Demandeur: L'OREAL, 75008 Paris (FR)
(72) Inventeur: Arnaud, Pascal, 94240 l'Hay les Roses (FR)
(74) Mandataire: Kromer, Christophe

(57) **Abrégé**

La présente invention se rapporte à une composition cosmétique de soin ou de maquillage des matières kératiniques comprenant, dans un milieu physiologiquement acceptable, au moins un agent photoprotecteur capable de filtrer le rayonnement UV et au moins un ester à groupement aromatique, liquide à température ambiante, résultant de l'estérification par un acide aromatique d'au moins un groupe hydroxyle pendant ou en bout de chaîne d'un composé aliphatique hydroxylé choisi parmi les acides aliphatiques hydroxylés et leurs esters.

L'invention se rapporte également à l'utilisation dudit ester à groupement aromatique pour améliorer le pouvoir photoprotecteur de compositions destinées à la protection de la peau et/ou des lèvres et/ou des phanères contre les rayonnements UV.

## Description

La présente invention se rapporte à une composition cosmétique de soin ou de maquillage destinée notamment à la protection des matières kératiniques d'êtres humains comme la peau et/ou les lèvres et/ou les phanères (ongles, cils, sourcils, poils, cheveux) contre le rayonnement ultraviolet (UV). Cette composition comprend en particulier un agent photoprotecteur capable de filtrer ou bloquer le rayonnement UV et un ester à groupement aromatique, liquide à température ambiante, résultant de l'estérification par un acide aromatique d'au moins un groupe hydroxyle d'un composé aliphatique hydroxylé choisi parmi les acides aliphatiques hydroxylés et leurs esters.

On sait que les rayonnements lumineux de longueurs d'onde comprises entre 280 nm et 400 nm permettent le brunissement de l'épiderme humain et que les rayons de longueurs d'onde comprises entre 280 et 320 nm, connus sous la dénomination d'UV-B, provoquent des érythèmes et des brûlures cutanées qui peuvent nuire au développement du bronzage naturel ; ce rayonnement UV-B doit donc être filtré.

On sait également que les rayons UV-A, de longueurs d'onde comprises entre 320 et 400 nm, qui provoquent le brunissement de la peau, sont susceptibles d'induire une altération de celle-ci, notamment dans le cas d'une peau sensible ou d'une peau continuellement exposée au rayonnement solaire. Les rayons UV-A provoquent en particulier une perte d'élasticité de la peau et l'apparition de rides conduisant à un vieillissement prématuré. Ils favorisent le déclenchement de la réaction érythémateuse ou amplifient cette réaction chez certains sujets et peuvent même être à l'origine de réactions phototoxiques ou photo-allergiques. Il est donc souhaitable de filtrer aussi le rayonnement UV-A.

Pour filtrer ou bloquer les rayons UVA et UVB, il existe sur le marché différents types d'agents photoprotecteurs solaires communément appelés filtres solaires, organiques ou inorganiques, solubles ou insolubles dans le milieu, capables d'absorber ou de bloquer sélectivement les rayonnements UV nocifs, ces filtres (et leurs quantités) étant sélectionnés en fonction du facteur de protection solaire recherché ; ce facteur de protection solaire (SPF) s'exprime mathématiquement par le rapport de la dose de rayonnement UV nécessaire pour atteindre le seuil érythématogène avec le filtre UV avec la dose de rayonnement UV nécessaire pour atteindre le seuil érythématogène sans filtre UV.

La formulation des compositions contenant des filtres solaires fait l'objet de nombreuses recherches dans le but d'améliorer l'efficacité des filtres organiques ou inorganiques (minéraux).

Il est bien connu que les huiles présentes dans les compositions solaires influencent les propriétés d'absorption des filtres et les valeurs de SPF. Le formulateur recherche ainsi des huiles qui présentent à la fois de très bonnes propriétés de solubilisation ou de dispersion des filtres organiques et minéraux, mais aussi de bonnes propriétés sensorielles, en particulier, d'application.

Par « huile », on entend tout composé non aqueux, liquide à température ambiante (25°C) et pression atmosphérique (760 mm de Hg).

Les huiles ayant dans leur structure chimique un radical aromatique constituent, selon les inventeurs, une famille privilégiée de solvants de filtres organiques dans la mesure où leur analogie de structure avec ces filtres organiques leur confère de très bonnes propriétés de solubilisation.

On peut citer, par exemple, le benzoate d'alkyle en C₁₂₋₁₅ (comme le FINSOLV TN commercialisé par la société FINETEX), les esters benzoïques d'alcools ramifiés comme ceux décrits dans la demande EP-A-848 944 de C.P. HALL Company, ou encore les silicones phénylées décrites dans le brevet US-A-5 008 101 de DOW CORNING.

Ces solvants ont des propriétés sensorielles qui sont tout à fait adaptées à la réalisation de produits cosmétiques caractérisés à l'application par un toucher sec, évanescent, et par l'obtention d'un film peu brillant, comme les produits solaires, les crèmes de soin, ou les lotions.

En revanche, ces solvants ne conviennent pas toujours pour la formulation d'autres produits, en particulier dans le domaine du maquillage comme les rouges à lèvres, les fonds de teint coulés ou dans le domaine du soin comme les crèmes nourrissantes ou les baumes de soin pour les lèvres.

On connaît d'autre part les esters aromatiques obtenus par la réaction de l'acide benzoïque sur des esters d'acides hydroxylés, en particulier l'huile de ricin, décrits dans le brevet US-A-5 959 130 de FINETEX.

A la suite de recherches approfondies menées sur ces esters, le demandeur a trouvé, de façon surprenante, qu'il était possible d'augmenter substantiellement le pouvoir photoprotecteur d'une composition destinée à la protection contre le rayonnement ultraviolet en associant au sein de cette composition un filtre UV et une huile spécifique qui est un ester à groupement aromatique, liquide à température ambiante, résultant de l'estérification par un acide aromatique d'au moins un groupe hydroxyle pendant ou en bout de chaîne d'un composé aliphatique hydroxylé choisi parmi les acides aliphatiques hydroxylés et leurs esters.

L'invention a donc pour objet une composition cosmétique de soin ou de maquillage des matières kératiniques comprenant, dans un milieu physiologiquement acceptable, au moins un agent photoprotecteur capable de filtrer le rayonnement UV, une phase particulaire et au moins un ester à groupement aromatique, liquide à température ambiante, résultant de l'estérification par un acide aromatique d'au moins un groupe hydroxyle pendant ou en bout de chaîne d'un composé aliphatique hydroxylé choisi parmi les acides aliphatiques hydroxylés et leurs esters. L'agent photoprotecteur, la phase particulaire et l'ester à groupement aromatique de la composition selon l'invention sont des composés distincts.

La présente invention a également pour objet une composition cosmétique de soin ou de maquillage des matières kératiniques comprenant, dans un milieu physiologiquement acceptable, au moins un agent photoprotecteur capable de filtrer le rayonnement UV contenant un ou plusieurs filtres particulaires inorganiques et au moins un ester à groupement aromatique, liquide à température ambiante, résultant de l'estérification par un acide aromatique d'au moins un groupe hydroxyle pendant ou en bout de chaîne d'un composé aliphatique hydroxylé choisi parmi les acides aliphatiques hydroxylés et leurs esters.

Il est certes décrit dans le brevet US-A-5 959 130 de FINETEX que ces esters à groupement aromatiques ont de bonnes propriétés de dispersion des pigments et de bonnes propriétés de solubilisation des filtres organiques solides. Cependant, ce document ne décrit pas des compositions solaires selon la présente invention et il ne mentionne ni ne suggère la capacité de ces esters à augmenter significativement le SPF de telles compositions.

La composition selon la présente invention est de préférence sous forme d'une composition destinée à la protection de la peau et/ou des lèvres et/ou des phanères contre le rayonnement UV, en particulier le rayonnement solaire.

L'invention a encore pour objet l'utilisation d'au moins un ester à groupement aromatique, liquide à température ambiante, résultant de l'estérification par un acide aromatique d'au moins un groupe hydroxyle pendant ou en bout de chaîne d'un composé aliphatique hydroxylé choisi parmi les acides aliphatiques hydroxylés et leurs esters, dans une composition cosmétique ou pour la fabrication d'une composition physiologique destinée à la protection de la peau et/ou des lèvres et/ou des phanères contre le rayonnement UV contenant au moins un agent photoprotecteur, pour améliorer le pouvoir photoprotecteur de ladite composition.

Par « composition physiologique », on entend une composition cosmétique ou dermatologique non toxique et susceptible d'être appliqué sur la peau, les phanères et/ou les lèvres du visage d'êtres humains.

L'invention a aussi pour objet l'utilisation d'au moins un ester à groupement aromatique, liquide à température ambiante, résultant de l'estérification par un acide aromatique d'au moins un groupe hydroxyle pendant ou en bout de chaîne d'un composé aliphatique hydroxylé choisi parmi les acides aliphatiques hydroxylés et leurs esters, dans une composition cosmétique solaire ou pour la fabrication d'une composition physiologique solaire contenant au moins un agent photoprotecteur.

Un autre objet de l'invention est un procédé cosmétique de protection de la peau, des lèvres et/ou des phanères d'êtres humains contre le rayonnement solaire, consistant à introduire, dans une composition cosmétique contenant au moins un agent photoprotecteur, une quantité efficace d'un ester à groupement aromatique, liquide à température ambiante, résultant de l'estérification par un acide aromatique d'au moins un groupe hydroxyle pendant ou en bout de chaîne d'un composé aliphatique hydroxylé choisi parmi les acides aliphatiques hydroxylés et leurs esters.

L'invention a encore pour objet un procédé pour augmenter les propriétés de protection solaire d'une composition cosmétique contenant au moins un agent photoprotecteur consistant à introduire dans ladite composition une quantité efficace d'un ester à groupement aromatique, liquide à température ambiante, résultant de l'estérification par un acide aromatique d'au moins un groupe hydroxyle pendant ou en bout de chaîne d'un composé aliphatique hydroxylé choisi parmi les acides aliphatiques hydroxylés et leurs esters.

L'invention a enfin pour objet un procédé pour augmenter le facteur de protection solaire d'une composition cosmétique contenant au moins un agent photoprotecteur consistant à introduire dans ladite composition une quantité efficace d'un ester à groupement aromatique, liquide à température ambiante, résultant de l'estérification par un acide aromatique d'au moins un groupe hydroxyle pendant ou en bout de chaîne d'un composé aliphatique hydroxylé choisi parmi les acides aliphatiques hydroxylés et leurs esters.

En plus de l'augmentation du facteur de protection, l'ester à groupement aromatique selon l'invention confère à la composition de bonnes propriétés sensorielles d'étalement et de glissant et confère également au film déposé des propriétés de bonne tenue dans le temps, de non collant et de non gras. La composition obtenue présente en outre une bonne homogénéité et une bonne stabilité thermique (non demixion de la composition ). L'obtention d'une bonne homogénéité du dépôt est particulièrement recherchée pour un produit de maquillage.

Par « agent photoprotecteur » au sens de la présente invention, on entend tout composé ou toute association de composés qui, par des mécanismes d'absorption et/ ou de réflexion et/ ou de diffusion du rayonnement UV-A et/ ou UV-B, permet d'empêcher, ou du moins limiter, la mise en contact dudit rayonnement avec la surface de la peau, des lèvres et/ou des phanères sur laquelle ce ou ces composés ont été appliqués.

Ces composés peuvent être des filtres organiques hydrophiles et/ou des filtres organiques lipophiles actifs dans l'UV-A et /ou l'UV-B et/ou des filtres inorganiques tels que des pigments ou des nano pigments minéraux diffuseurs et/ ou réflecteurs d'UV, ainsi que leurs mélanges.

L'agent photoprotecteur peut représenter de 0,1 à 45% et de préférence de 0,5 à 25% du poids total de la composition.

De préférence, l'agent photoprotecteur contient un ou plusieurs filtres particulaires à savoir un ou plusieurs filtres insolubles à température ambiante dans le milieu physiologiquement acceptable de la composition. En particulier, ces filtres particulaires sont des filtres inorganiques ou minéraux.

De préférence, le ou les filtres particulaires inorganiques sont choisis parmi les pigments ou les nano pigments d'oxydes métalliques, traités ou non, capables de bloquer physiquement, par diffusion et/ou réflexion, le rayonnement UV.

Par pigments ou nano pigments traités, on entend des pigments qui ont subi un ou plusieurs traitements de surface de nature chimique, électronique, mécanochimique et/ou mécanique avec des composés tels que décrits par exemple dans Cosmetics & Toiletries, Février 1990, Vol. 105, p 53-64, tels que des aminoacides, de la cire d'abeille, des acides gras, des alcools gras, des tensio-actifs anioniques, des lécithines, des sels de sodium, potassium, zinc, fer, ou aluminium d'acides gras, des alcoxydes métalliques (de titane ou d'aluminium), du polyéthylène, des silicones, des protéines (collagène, élastine), des alcanolamines, des composés fluorés, des oxydes de silicium, des oxydes métalliques, de l'hexamétaphosphate de sodium, de l'alumine ou de la glycérine.

Selon un mode préféré de réalisation de l'invention, les filtres particulaires inorganiques sont choisis parmi les nano pigments d'oxydes de titane (amorphe ou cristallisé sous forme rutile et/ou anatase), de zinc, de fer, de zirconium, de cérium, traités ou non, et leurs mélanges.

Les nano pigments traités peuvent être plus particulièrement des nano oxydes de titane traités par :
- la silice et l'alumine tels que les produits « Microtitanium Dioxide MT 500 SA » et « Microtitanium Dioxide MT 100 SA » de la société TAYCA, et les produits « Tioveil Fin », « Tioveil OP », « Tioveil MOTG » et « Tioveil IPM » de la société TIOXIDE,
- l'alumine et le stéarate d'aluminium tels que le produit « Microtitanium Dioxide MT 100 T » de la société TAYCA,
- l'alumine et le laurate d'aluminium tels que le produit « Microtitanium Dioxide MT 100 S » de la société TAYCA,
- des oxydes de fer et le stéarate de fer tels que le produit « Microtitanium Dioxide MT 100 F » de la société TAYCA,
- la silice, l'alumine et la silicone tels que les produits « Microtitanium Dioxide MT 100 SAS », « Microtitanium Dioxide MT 600 SAS » et « Microtitanium Dioxide MT 500 SAS » de la société TAYCA,
- l'hexamétaphosphate de sodium tels que le produit « Microtitanium Dioxide MT 150 W » de la société TAYCA,
- l'octyltriméthoxysilane tels que le produit « T-805 » de la société DEGUSSA,
- l'alumine et l'acide stéarique tels que le produit « UVT-M160 » de la société KEMIRA,
- l'alumine et la glycérine tels que le produit « UVT-M212 » de la société KEMIRA,
- l'alumine et la silicone tels que le produit « UVT-M262 » de la société KEMIRA.

Les nano oxydes de titane non traités peuvent par exemple être ceux vendus par la société TAYCA sous les dénominations commerciales « Microtitanium Dioxide MT 500 B » ou « Microtitanium Dioxide MT 600 B ».

Les nano oxydes de zinc non traités peuvent par exemple être ceux vendus par la société SUMITOMO sous la dénomination « Ultra Fine Zinc Oxide Powder », par la société PRESPERSE sous la dénomination « Finex 25 », par la société IKEDA sous la dénomination « MZO-25 » ou par la société SUNSMART sous la dénomination « Z-COTE ». Les nano oxydes de zinc traités peuvent par exemple être ceux vendus par la société SUNSMART sous la dénomination « Z-COTE HP 1 ».

Les nano pigments peuvent être introduits dans les compositions selon l'invention tels quels ou sous forme de pâte pigmentaire, c'est-à-dire en mélange avec un dispersant, comme décrit par exemple dans le document GB-A-2206339.

De préférence, on choisit des nano pigments contenant des particules élémentaires dont la taille est comprise entre 5 et 500 nm, de préférence entre 10 et 250 nm, et mieux de 10 à 100 nm, par exemple de 10 à 50 nm.

Les nanopigments peuvent représenter de 0,1 à 30 % et de préférence de 0,5 à 15 % du poids total de la composition selon l'invention.

Les filtres organiques, actifs dans l'UV-A et/ou l'UV-B, hydrophiles ou lipophiles peuvent être choisis notamment parmi les dérivés cinnamiques; les dérivés de dibenzoylméthane ; les dérivés salicyliques (filtres lipophiles), les dérivés du camphre ; les dérivés de triazine (filtres lipophiles) tels que ceux décrits dans les demandes de brevet US 4367390, EP863145, EP517104, EP570838, EP796851, EP775698, EP878469 et EP933376 ; les dérivés de la benzophénone ; les dérivés de β,β'-diphénylacrylate, les dérivés de benzimidazole ; les dérivés bis-benzoazolyle tels que ceux décrits dans les brevets EP-A-0669323 et US 2,463,264 ; les dérivés de bis-hydroxyphénolbenzotriazole tels que ceux décrits dans les demandes US 5237071, US 5166355, GB-A-2303549, DE 19726184 et EP-A-893119 ; les dérivés de l'acide p-aminobenzoïque (filtres hydrophiles); les polymères hydrocarbonés filtres, les silicones filtres lipophiles tels que ceux décrits notamment dans la demande WO-93/04665 et leurs mélanges.

Comme exemples de filtres organiques lipophiles actifs dans l'UV-A et/ou l'UV-B, on peut citer :
l'acide p-aminobenzoïque,
le p-aminobenzoate oxyéthyléné (25mol),
le p-diméthylaminobenzoate de 2-éthylhexyle,
le p-aminobenzoate d'éthyle N-oxypropyléné,
le p-aminobenzoate de glycérol,
le salicylate d'homomenthyle,
le salicylate de 2-éthylhexyle,
le salicylate de triéthanolamine,
le salicylate de 4-isopropylbenzyle,
le 4-ter-butyl-4'-méthoxy-dibenzoylméthane,
le 4-isopropyl-dibenzoylméthane,
le 4-méthoxy cinnamate de 2-éthylhexyle,
le diisopropyl cinnamate de méthyle,
le 4-méthoxy cinnamate d'isoamyle,
le 4-méthoxy cinnamate de diéthanolamine,
l'anthranilate de menthyle,
le 2-éthylhexyl-2-cyano-3,3'-diphénylacrylate,
l'éthyl-2-cyano-3,3'-diphénylacrylate,
le 2-hydroxy-4-méthoxybenzophénone,
le 2,4-dihydroxybenzophénone,
le 2,2',4,4'-tétrahydroxybenzophénone,
le 2,2'-dihydroxy-4,4'diméthoxybenzophénone,
le 2-hydroxy-4-n-octoxybenzophénone,
le 2-hydroxy-4-méthoxy-4'-méthylbenzophénone,
le 3-(4'méthylbenzylidène)-d,l-camphre,
le 3-benzylidène-d,l-camphre,
l'acide urocanique,
la 2,4,6-tris-[p-(2'-éthylhexyl-1'-oxycarbonyl)anilino]-1,3,5-triazine,
la 2-[p-(tertiobutylamido)anilino]-4,6-bis-[(p-(2'-éthylhexyl-1'-oxycarbonyl)anilino]-1,3,5-triazine,
la 2,4-bis {[4-2-éthyl-hexyloxy)]-2-hydroxy]-phenyl}-6-(4-méthoxy-phenyl)-1,3,5-triazine ;
le polymère de N-(2 et 4)-[(2-oxoborn-3-ylidèn)méthyl] benzyl]-acrylamide,
les polyorganosiloxanes à fonction benzalmalonate,
les polyorganosiloxanes à fonction benzotriazole tel que le Drometrizole Trisiloxane,
et leurs mélanges.

Comme filtres organiques hydrophiles actifs dans l'UV-A et/ou l'UV-B, utilisables dans la présente invention, on peut citer :
l'acide 2-phényl benzimidazole 5-sulfonique et ses sels,
l'acide α-(2-oxoborn-3-ylidène)-tolyl-4-sulfonique et ses sels solubles,
le 2-hydroxy-4-méthoxybenzophénone-5-sulfonate,
le 3-(4'-triméthylammonium)-benzylidèn-bornan-2-on-méthylsulfate,
le 3-(4'-sulfo)benzylidèn-bornan-2-one et ses sels solubles,
l'acide 1,4-bis-benzimidazolyl-phénylen-3,3',5,5'-tétrasulfonique et ses sels solubles,
l'acide benzène 1,4-di(3-méthylidène-10-camphosulfonique) et ses sels solubles,
et leurs mélanges.

Comme filtres organiques insolubles dans le milieu, on peut citer le 2,2'-méthylène-bis-[6-(2H-benzotriazol-2-yl)-4-(1,1,3,3-tétraméthylbutyl)phénol] ou le composé (2,2'-méthylène-bis-[6-(2H-benzotriazol-2-yl)-4-(methyl)phénol].

Comme filtres organiques lipophiles particulièrement utilisables dans la présente invention, on peut citer :
- le 4-tert-butyl-4'-méthoxydibenzoylméthane vendu sous la dénomination commerciale «PARSOL 1789» par la société HOFFMANN LAROCHE ;
- l'octylméthoxycinnamate vendu sous la dénomination commerciale «PARSOL MCX» par la société HOFFMANN LAROCHE ;
- l'α-cyano- β,β -diphénylacrylate de 2-éthylhexyle (octocrylène) vendu sous la dénomination commerciale «UVINUL N 539» par la société BASF ;
- le 4-méthylbenzylidène camphre vendu sous la dénomination commerciale «EUSOLEX 6300» par MERCK ;
- la benzophénone-3 (oxybenzone) vendue sous la dénomination commerciale «UVINUL M40» par BASF ;
- le salicylate de 2-éthylhexyle ou octylsalicylate vendue sous la dénomination commerciale NEO HELIOPAN OS par HAARMAAN & REIMER ;
- la 2,4,6-tris[p-(2'-éthylhexyl-1'-oxycarbonyl)anilino]-1,3,5-triazine vendue sous la dénomination «UVINUL T 150» par la Société BASF;
- le Drometrizole Trisiloxane vendu sous la dénomination commerciale «SILATRIZOLE» par la société RHODIA
- et leurs mélanges.

Comme filtres organiques hydrophiles particulièrement utilisables dans la présente invention, on peut citer l'acide benzène 1,4-di(3-méthylidène-10-campho-sulfonique) vendue sous le nom commercial «MEXORYL SX» par CHIMEX et l'acide 2-phénylbenzimidazole-5-sulfonique vendu sous la dénomination commerciale «EUSOLEX 232» par la société MERCK.

De préférence, on choisit un ou des filtres organiques solubles dans le milieu physiologiquement acceptable de la composition selon l'invention.

Les filtres organiques peuvent représenter de 0,1 à 15 %, et mieux de 1 à 10% du poids total de la composition selon l'invention.

La composition selon l'invention contient également un ou plusieurs esters à groupement aromatique, liquide à température ambiante, résultant de l'estérification par un acide aromatique d'au moins un groupe hydroxyle pendant ou en bout de chaîne d'un composé aliphatique hydroxylé choisi parmi les acides aliphatiques hydroxylés et leurs esters.

Par composé aliphatique hydroxylé, on entend un acide hydroxy carboxylique aliphatique ou un ester d'acide hydroxy carboxylique aliphatique. L'acide (non estérifié) comporte notamment de 2 à 40 atomes de carbone, de préférence de 10 à 34 atomes de carbone et mieux de 12 à 28 atomes de carbone ; il comporte, en outre, de 1 à 20 groupes hydroxyle, de préférence de 1 à 10 groupes hydroxyle et mieux de 1 à 6 groupes hydroxyle, susceptible(s) d'être estérifié(s) par l'acide aromatique. Le composé hydroxylé sous forme d'ester résulte de l'estérification de la fonction -COOH d'un acide aliphatique hydroxy carboxylique par un alcool aliphatique qui peut comprendre de 1 à 40 atomes de carbone et mieux de 3 à 30 atomes de carbone. Cet alcool peut être un monoalcool ou un polyol. Les esters issus de la réaction de l'acide hydroxy carboxylique aliphatique avec un polyol peuvent être des esters partiellement ou totalement estérifiés.

De préférence le composé aliphatique hydroxylé est choisi parmi les esters issus d'acide hydroxy carboxylique aliphatique. Autrement dit, l'ester à groupement aromatique liquide de la composition de l'invention est un ester d'ester. Avantageusement, cet ester à groupement aromatique est un ester d'ester d'acide gras dont le reste d'acide gras comporte au moins 12 atomes de carbone. De préférence, le groupe hydroxyle engagé dans l'estérification par l'acide aromatique est porté sur la partie acide du composé hydroxylé.

Selon l'invention, la composition peut contenir un ou plusieurs esters à groupement aromatique, liquides à température ambiante (25 °C) et pression atmosphérique (760 mm de Hg).

Lorsque le groupe hydroxyle du composé aliphatique hydroxylé engagé dans l'estérification avec l'acide aromatique est en bout de chaîne, ce groupe est en position α-ω par rapport à la fonction -COOH de l'acide hydroxy carboxylique aliphatique.

L'acide aromatique peut être choisi parmi les acides carboxyliques suivants :
a) les mono acides, tels que l'acide benzoïque, l'acide phényl acétique, l'acide cinnamique, l'acide 3 phényl propanoïque, l'acide salicylique ;
b) les diacides tels que l'acide téréphtalique ;
c) les triacides tels que l'acide trimellitique ;
d) les tétra-acides tels que l'acide pyromellitique et leurs mélanges.
De préférence, l'acide aromatique est non hydroxylé (en particulier le groupement aromatique n'est pas hydroxylé).
De façon avantageuse, l'acide aromatique est l'acide benzoïque.

Comme ester utilisable dans l'invention, on peut citer ceux résultant de l'estérification par au moins un acide aromatique de l'un au moins des acides carboxyliques hydroxylés aliphatiques suivants :
i) les monoacides aliphatiques mono hydroxylés linéaires saturés de formule : 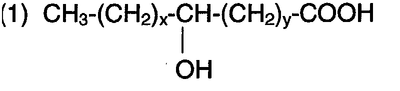 avec 0 ≤ x + y ≤ 37
   comme l'acide lactique (x + y = 0) ; l'acide 12-hydroxy octadécanoïque (ou 12-hydroxy stéarique) de formule : 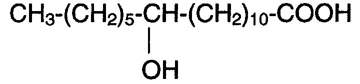 avec x = 5, y = 10 et x + y = 15
   et l'acide α-hydroxy octadécanoïque 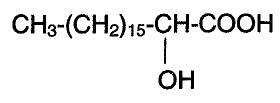 (avec x = 5 et y = 0) ou

   (2) HO-CH₂-(CH₂)ₓ-COOH

   avec 0 ≤ x ≤ 38
   comme l'acide glycolique HO-CH₂-COOH (x = 0) ; ou l'acide junipérique (acide 16-hydroxy hexadécanoïque) de formule HO-CH₂-(CH₂)₁₄-COOH avec x = 14 ;
ii) les monoacides aliphatiques mono hydroxylés ramifiés saturés de formule : 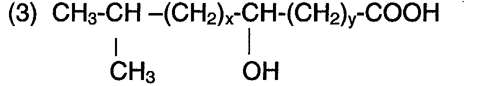 avec 0 ≤ x + y ≤ 35 comme l'acide 5-méthyl 2-hydroxy hexanoïque (acide leucinique) de formule : 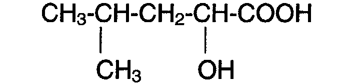 avec x = 1 y = 0 et x + y = 1
   ou (3') encore l'acide 2-éthyl 3-hydroxy caprylique de formule : 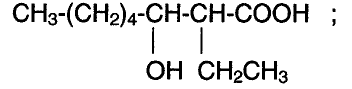
iii) Les monoacides aliphatiques mono hydroxylés insaturés de formule : 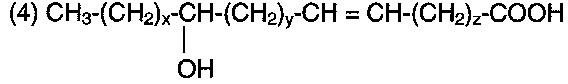 avec 0 ≤ x + y + z ≤ 35
   comme l'acide 12-hydroxy (cis) 9-octadécénoïque (ou l'acide ricinoléique) de formule : 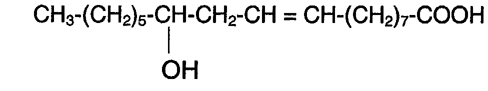 avec x = 5, y = 1, z = 7 et x + y + z = 13
   ou 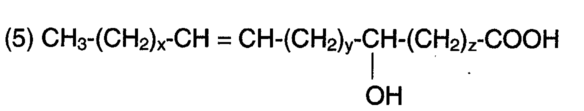 avec 0 ≤ x + y + z ≤ 35
   comme l'acide 3-hydroxy 4-hexanoïque de formule 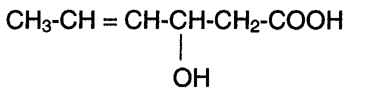 avec x = 0, y =0, z = 1 et x + y + z = 1
   ou l'acide 2-hydroxy 15-tétracosènoïque (ou acide oxynervonique) de formule : 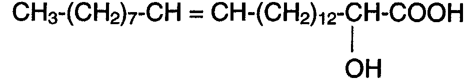 avec x = 7, y = 12, z = 0 et x + y + z = 17
   ou

   (6) HOCH₂-(CH₂)ₓ-CH = CH-(CH₂)_{y}-COOH

   avec 0 ≤ x + y ≤ 36
   comme l'acide 16-hydroxy 6-hexadécènoïque avec x = 8, y = 4 et x + y = 12 de formule HO-CH₂-(CH₂)₈-CH = CH-(CH₂)₄-COOH ;
iv) les monoacides aliphatiques poly hydroxylés saturés de formule : 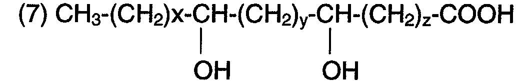 avec 0 ≤ x + y + z ≤ 36
   comme l'acide 9, 10-dihydroxy octadécanoïque de formule 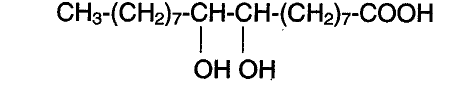 avec x = 7, y = 0, z = 7 et x + y + z = 14
   comme l'acide 9, 12-dihydroxy octadécanoïque de formule 7 avec x = 5, y = 2, z = 7 et x + y + z = 14
   ou l'acide 9, 10, 16-trihydroxy hexadécanoïque (acide aleuritique), l'acide 9, 10, 12- trihydroxy octadécanoïque de formule: 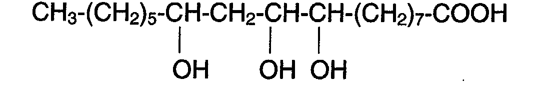 ou encore l'acide hexahydroxy octadécanoïque et l'acide octahydroxy octadécanoïque ;
v) les polyacides aliphatiques mono hydroxylés saturés de formule : 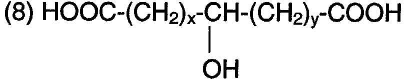 avec 0 ≤ x + y ≤ 37 comme l'acide malique, ou encore l'acide citrique ; et
vi) les polyacides aliphatiques poly hydroxylés saturés comme l'acide tartrique ;
et leurs mélanges.

Comme autre composé hydroxylé aliphatique utilisable dans l'invention, on peut citer ceux résultant de l'estérification par au moins un acide aromatique de l'un au moins des esters d'acides hydroxylés aliphatiques suivants :
vii) les esters de monoacides aliphatiques mono hydroxylés linéaires saturés tels que :
   - les esters de l'acide lactique comme le lactate d'isostéaryle, le lactate issu d'alcool en C₁₂-C₁₃, le lactate d'octyl dodécyle, le lactate d'oléyle, le lactate de myristyle ;
   - les esters de l'acide 12-hydroxy octadécanoïque (ou 12-hydroxy stéarique) comme l'hydroxy stéarate d'éthyl 2-hexyle, l'hydroxy stéarate d'octyl docécyle, l'hydroxy stéarate d'isostéaryle, l'hydroxy stéarate d'isodécyle, le trihydroxy stéarate de glycéryle (ou huile de ricin hydrogénée), l'hexahydroxy stéarate de dipentaérythrityle ;
viii) les esters de monoacides aliphatiques mono hydroxylés insaturés tels que les esters de l'acide ricinoléique (ou acide 12-hydroxy (cis) 9-octadécénoïque) comme le ricinoléate de butyle, le ricinoléate d'octyl dodécyle, le ricinoléate de cétyle, le triricinoléate de glycéryle, l'huile de ricin ;
ix) les esters de polyacides aliphatiques mono hydroxylés saturés tels que le malate de diisostéaryle, le citrate de triisostéaryle, le citrate de trioctyl dodécyle ;
x) les esters de polyacides aliphatiques poly hydroxylés saturés comme le tartrate issu de la réaction avec 2 alcools en C₁₂-C₁₃ ramifiés ;
et leurs mélanges.

Comme composé hydroxylé sous forme d'ester, utilisable dans l'invention et résultant de l'estérification d'un polyol, on peut citer de façon générale :
xi) les esters partiels ou totaux de polyol en C₂ à C₁₆ ayant réagit avec un acide aliphatique hydroxylé comme notamment les triglycérides, les esters du pentaérythritol, du néopentyl glycol, du dipentaérythritol, du polyglycérol, les esters du sorbitol ;
et leurs mélanges.

De préférence, les esters à groupement aromatique de l'invention sont choisis parmi les esters d'ester d'acides gras aliphatiques dont le reste d'acide gras comporte au moins 12 atomes de carbone. En particulier, le composé hydroxylé est choisi parmi les esters de l'acide ricinoléique, les esters de l'acide 12-hydroxy stéarique, les esters de l'acide lactique, les esters de l'acide hydroxy 14-eicosènoïque et leurs mélanges.

Comme ester à groupement aromatique, on utilise notamment :
- l'ester résultant de la réaction d'estérification de l'huile de ricin avec l'acide benzoïque dans les proportions de 1 pour 1 (1/1) par exemple commercialisé par la société FINETEX sous la référence FINSOLV BCO-110 et qui sera appelé par la suite glyceryl monobenzoyl ricinoleate,
- le composé résultant de la réaction de l'huile de ricin avec l'acide benzoïque dans les proportions de 1 pour 1,5 (1/1,5) par exemple commercialisé par la société FINETEX sous la référence FINSOLV BCO-115 et qui sera appelé par la suite glyceryl mono -dibenzoyl ricinoleate,
- le composé résultant de la réaction d'estérification de l'huile de ricin avec l'acide benzoïque dans les proportions de 1 pour 2 (1/2) par exemple commercialisé par la société FINETEX sous la référence FINSOLV BCO-120 et qui sera appelé par la suite glyceryl dibenzoyl ricinoleate,
- le composé résultant de la réaction d'estérification de l'huile de ricin avec l'acide benzoïque dans les proportions de 1 pour 3 (1/3) par exemple commercialisé par la société FINETEX sous la référence FINSOLV BCO-130 et qui sera appelé par la suite glyceryl tribenzoyl ricinoleate, et
- leurs mélanges.

De préférence, l'ester à groupement aromatique utilisé est le glyceryl mono-dibenzoyl ricinoleate.

L'acide ricinoléique représente de 80 à 92 % de l'huile de ricin. Ainsi son estérification conduit majoritairement (80 à 92 %) à l'ester de l'ester de l'acide ricinoléique.

On peut aussi utiliser l'ester résultant de la réaction d'estérification avec de l'acide benzoïque de l'huile de lesquerella contenant majoritairement (52 à 57 %) un ester de l'acide hydroxy 14-eicosènoïque ou acide lesquerolique.

L'ester à groupement aromatique de la composition de l'invention peut être fabriqué selon le procédé décrit dans le document US-A-5 959 130.

L'ester à groupement aromatique de la composition de l'invention peut représenter de 0,1 à 99,9 % du poids total de la composition, de préférence de 1 à 99 % et mieux de 5 à 90%, par exemple de 5 à 70%.

Avantageusement, le rapport ester à groupement aromatique/agent photoprotecteur dans les compositions selon l'invention peut aller de 999 à 0,01, et mieux de 200 à 1.

Selon un mode préféré de réalisation, la composition selon l'invention comprend une phase particulaire. Par « phase particulaire », on entend une phase contenant une ou plusieurs particules solides à température ambiante, amorphes ou cristallines, insolubles dans le milieu à température ambiante et même au-dessus de leur température de fusion ou de ramollissement.
Cette phase particulaire peut être présente à raison de 0,01 à 40 % du poids total de la composition, de préférence de 0,5 à 25 %.

Elle peut comprendre des pigments et/ou des nacres et/ou des charges habituellement utilisés dans les compositions cosmétiques. Les pigments peuvent être blancs ou colorés, minéraux ou organiques, enrobés ou non, de taille usuelle ou nanométrique, ils sont insolubles dans le milieu physiologiquement acceptable de la composition selon l'invention. Ils sont destinés à colorer et/ou opacifier la composition. Ces pigments peuvent être des filtres particulaires inorganiques (ou minéraux) tels que les pigments ou les nano pigments minéraux diffuseurs et/ ou réflecteurs d'UV précédemment décrits. On peut citer, parmi les pigments minéraux, les dioxydes de titane, de zinc, de zirconium ou de cérium, ainsi que les oxydes de zinc, de fer ou de chrome, le bleu ferrique, l'hydrate de chrome, les outremers (polysulfures d'aluminosilicates), le pyrophosphate de manganèse et certaines poudres métalliques telles que celles d'argent ou d'aluminium, Parmi les pigments organiques, on peut citer le noir de carbone, et les laques à base de carmin de cochenille, de baryum, strontium, calcium, aluminium. Les pigments peuvent être présents dans la composition à raison de 0,05 à 40 % du poids total de la composition, et de préférence à raison de 2 à 20 %.

La phase particulaire peut également comprendre des filtres organiques insolubles dans le milieu tels que les filtres cités précédemment.

Par « nacres » ou « pigments nacrés » il faut comprendre des particules irisées, notamment produites par certains mollusques dans leur coquille ou bien synthétisées, insolubles dans le milieu physiologiquement acceptable de la composition.
Les nacres peuvent être présentes dans la composition à raison de 0,01 à 20% en poids, de préférence de 1 à 15% en poids, et peuvent être choisies parmi les pigments nacrés blancs tels que le mica recouvert de titane, ou d'oxychlorure de bismuth, les pigments nacrés colorés tels que le mica titane avec des oxydes de fer, le mica titane avec notamment du bleu ferrique ou de l'oxyde de chrome, le mica titane avec un pigment organique du type précité.

De préférence, la composition selon l'invention contient des pigments et/ou des nacres, ces composés étant distincts du ou des agents photoprotecteurs présents dans ladite composition.

Les charges peuvent être choisies parmi le talc, le mica, la silice, le kaolin, les poudres de polyamide, de poly-β-alanine et de polyéthylène, les poudres de polymères de tétrafluoroéthylène, la lauroyl-lysine, l'amidon, le nitrure de bore, les microsphères polymériques creuses, les particules de polymère acrylique, les microbilles de résine de silicone, le carbonate de calcium précipité, le dicalcium phosphate, le carbonate et l'hydro-carbonate de magnésium, l'hydroxyapatite, les microsphères de silice creuses, les microcapsules de verre ou de céramique, les savons métalliques dérivés d'acides organiques carboxyliques ayant de 8 à 22 atomes de carbone, de préférence de 12 à 18 atomes de carbone et leurs mélanges. Les charges peuvent être présentes à raison de 0,01 à 35% en poids, de préférence 0,5 à 15%, dans la composition.

La composition peut en outre contenir une phase aqueuse comprenant de l'eau et éventuellement un ou plusieurs composés solubles tout ou partie dans l'eau.

La composition de l'invention peut comprendre, en plus de l'ester à groupement aromatique, au moins un corps gras additionnel choisi parmi les cires, les corps gras liquides à température ambiante (25°C) et pression atmosphérique (760 mm de Hg) différents dudit ester à groupement aromatique, appelés aussi huiles, les corps gras pâteux à température ambiante, les gommes, les résines, les polymères lipophiles et leurs mélanges.

Ces corps gras additionnels peuvent représenter de 0,01 à 90% du poids total de la composition, de préférence, de 0,05 à 60% et mieux de 1 à 35%.

Les huiles peuvent être des huiles hydrocarbonées et/ou siliconées et/ou fluorées. Ces huiles peuvent être d'origine animale, végétale, minérale ou synthétique. Par « huile hydrocarbonée », on entend une huile comportant principalement des atomes de carbone et d'hydrogène et éventuellement une ou plusieurs fonctions choisies parmi les fonctions hydroxyle, ester, éther, carboxylique. A titre d'exemple d'huile utilisable dans l'invention, on peut citer :
- les huiles hydrocarbonées d'origine animale telles que le perhydrosqualène ;
- les huiles hydrocarbonées végétales telles que les triglycérides liquides d'acides gras de 4 à 24 atomes de carbone comme les triglycérides des acides heptanoïque ou octanoïque ou encore les huiles de tournesol, de maïs, de soja, de courge, de pépins de raisin, de sésame, de noisette, d'abricot, de macadamia, de ricin, d'avocat, les triglycérides des acides caprylique/caprique comme ceux vendus par la société Stearineries Dubois ou ceux vendus sous les dénominations Miglyol 810, 812 et 818 par la société Dynamit Nobel, l'huile de jojoba, de beurre de karité ;
- les hydrocarbures linéaires ou ramifiés, d'origine minérale ou synthétique tels que les huiles de paraffine et leurs dérivés, la vaseline, les polydécènes, le polyisobutène hydrogéné tel que le Parleam ;
- les esters et les éthers de synthèse notamment d'acides gras comme les huiles de formule R₁COOR₂ dans laquelle R₁ représente le reste d'un acide gras supérieur comportant de 1 à 40 atomes de carbone et R₂ représente une chaîne hydrocarbonée contenant de 1 à 40 atomes de carbone avec R₁ + R₂ ≥ 10 comme par exemple l'huile de Purcellin, l'isononanoate d'isononyle, le myristate d'isopropyle, le palmitate d'éthyl 2-hexyle, le stéarate d'octyl 2-dodécyle, l'érucate d'octyl 2-dodécyle, l'isostéarate d'isostéaryle, le tridecyl trimellitate ; les esters hydroxylés comme l'isostéaryl lactate, l'octyl hydroxy stéarate, l'hydroxy stéarate d'octyl dodécyle, le diisostéaryl malate, le citrate de triisocétyle, des heptanoates, octanoates, décanoates d'alcools gras ; des esters de polyol comme le dioctanoate de propylène glycol, le diheptanoate de néopentyl glycol, le diisononanoate de diéthylène glycol ; et les esters du pentaérythritol comme le tétra-isostéarate de pentaérythrytyle ;
- des alcools gras ayant de 12 à 26 atomes de carbone comme l'octyl dodécanol, le 2-butyloctanol, le 2-hexyl décanol, le 2-undécyl pentadécanol, l'alcool oléique ;
- les huiles fluorées éventuellement partiellement hydrocarbonées et/ou siliconées ;
- les huiles siliconées comme les polydiméthylsiloxanes (PDMS) volatiles ou non, linéaires ou cycliques ; les polydiméthylsiloxanes comportant des groupements alkyle, alcoxy ou phényle, pendant ou en bout de chaîne siliconée, groupements ayant de 2 à 24 atomes de carbone ; les silicones phénylées comme les phényl triméthicones, les phényl diméthicones, les phényl triméthylsiloxy diphényl siloxanes, les diphényl diméthicones, les diphényl méthyldiphényl trisiloxanes, les 2-phényl éthyl triméthyl-siloxysilicates,
- leurs mélanges.

De préférence, la composition selon l'invention contient une cire. Par « cire », on entend un composé cristallin, soluble dans le milieu au-dessus de sa température de fusion et insoluble dans le milieu à température ambiante.

Comme cire utilisable dans l'invention, on peut citer celles généralement utilisées dans le domaine cosmétique : elles sont notamment d'origine naturelle éventuellement modifiée comme la lanoline, la lanoline oxypropylénée ou acétylée, la cire d'abeilles, la cire de Carnauba, de Candelilla, d'Ouricoury, du Japon, de fibres de liège ou de canne à sucre, de riz, de Montan, la paraffine, les cires de lignite ou microcristalline, la cérésine ou l'ozokérite, les huiles hydrogénées comme l'huile de jojoba ou l'huile de ricin hydrogénée ; les cires synthétiques comme les cires de polyéthylène issues de la polymérisation ou copolymérisation de l'éthylène et les cires de Fischer-Tropsch ou encore des esters d'acides gras comme l'octacosanyl stéarate, les glycérides concrets à 30°C et mieux à 45°C, les cires de silicones comme les alkyl- ou alkoxydiméthicones ayant une chaîne alkyle ou alcoxy de 10 à 45 atomes de carbone, les esters de poly(di)méthylsiloxane solide à 30°C dont la chaîne ester comporte au moins 10 atomes de carbone ; et leurs mélanges.

La composition selon l'invention peut également contenir un ou plusieurs additifs usuellement utilisés dans le domaine concerné tel que des adoucissants, des antioxydants, des conservateurs, des opacifiants, des neutralisants, des stabilisants, des émollients, des silicones, des composés fluorés, des agents anti-mousse, des parfums, des tensioactifs, des polymères, des propulseurs, des agents alcalinisants ou acidifiants, des gélifiants lipophiles ou de corps gras liquides, des gélifiants de phase aqueuse, des dispersants, des matières colorantes, des actifs cosmétiques. Ces additifs peuvent être présents dans la composition à raison de 0,001 à 30% du poids total de la composition et mieux de 0.005 à 15%.

Comme matières colorantes on peut citer les colorants hydrosolubles ou liposolubles, notamment les colorants organiques naturels tel que le carmin de cochenille, et/ou les colorants de synthèse tel que les colorants halogéno-acides, azoïques, anthraquinoniques. On peut également citer des colorants minéraux tels que le sulfate de cuivre.

Parmi les actifs cosmétiques pouvant être présents dans la composition, on peut citer les α-hydroxyacides, les agents répulsifs contre les insectes, les antiinflammatoires, les antagonistes de substance P. les vitamines A, E, C, B₃, les provitamines comme le D-panthénol, les actifs apaisants comme l'α-bisabolol l'aloe vera, l'allantoïne, les extraits de plantes ou les huiles essentielles, les agents protecteurs ou restructurants comme les céramides, les actifs fraîcheur comme le menthol et ses dérivés, les émollients (beurre de cacao, diméthicone), les hydratants (arginine PCA), le glycérol, les actifs antirides, les acides gras essentiels, et leurs mélanges.

Les compositions selon l'invention peuvent également contenir des agents de bronzage et/ou de brunissage artificiels de la peau (agents autobronzants), tels que par exemple de la dihydroxyacétone (DHA).

Bien entendu, l'homme du métier veillera à choisir la nature et la concentration de ces adjuvants de manière à ce qu'ils ne diminuent pas l'indice de protection de la composition et ne déstabilisent pas cette dernière.

Les compositions selon l'invention peuvent se présenter en particulier sous forme d'une solution huileuse, d'un gel huileux ou anhydre, d'une émulsion huile-dans-eau ou eau-dans-huile, d'une émulsion multiple, d'une dispersion d'huile dans de l'eau grâce à des vésicules.

Lorsqu'il s'agit d'une émulsion, la phase aqueuse de celle-ci peut comprendre une dispersion vésiculaire non ionique préparée selon des procédés connus (Bangham, Standish and Watkins. J. Mol. Biol. 13, 238 (1965), FR2315991 et FR2416008).

Dans un mode de réalisation particulier de l'invention, la composition selon l'invention peut être préparée de manière usuelle par l'homme du métier. Elle peut se présenter sous forme d'un produit coulé et par exemple sous la forme d'un stick ou bâton, sous forme de pâte souple ou encore de gel, de crème plus ou moins fluide, de lait, de pommade, de poudre, de mousse aérosol ou de spray.

De préférence, la composition selon l'invention se présente sous forme de stick plus ou moins rigide.

La composition cosmétique de l'invention peut être utilisée comme composition protectrice de l'épiderme humain ou des cheveux contre les rayons ultraviolets, comme composition solaire ou comme produit de maquillage.

Lorsque la composition cosmétique selon l'invention est utilisée pour la protection de l'épiderme humain contre les rayons UV, ou comme composition solaire, elle peut se présenter sous forme de forme d'un produit coulé, d'une pâte souple, d'une crème, d'un lait, d'une pommade, d'un gel, d'un gel crème, d'un stick ou d'un bâtonnet solide, d'une poudre, d'une mousse aérosol ou d'un spray.

Lorsque la composition cosmétique selon l'invention est utilisée pour la protection des cheveux contre les rayons UV, elle peut se présenter sous forme de shampooing, d'après shampooing, avant ou après coloration ou décoloration, avant, pendant ou après permanente ou défrisage, une lotion ou un gel coiffants ou traitants, une lotion ou un gel pour le brushing ou la mise en plis, une composition de permanente ou de défrisage, de coloration ou décoloration des cheveux.

La composition peut être utilisée comme composition de maquillage des cils, des sourcils, de la peau ou des lèvres, tel que crème de traitement de l'épiderme, fond de teint, bâton de rouge à lèvres, brillant à lèvres, produit ayant des propriétés de soin tel qu'un baume à lèvres, fard à paupières, fard à joues, mascara ou eye liner, produit de coloration de la peau (tel qu'un autobronzant).

De préférence, la composition selon l'invention est une composition de maquillage des lèvres.

Selon un mode préféré de réalisation, la composition se présente sous forme anhydre (moins de 5% d'eau).

Avantageusement, la composition est sous forme solide à température ambiante.

Les exemples qui suivent ont pour but d'illustrer de manière non limitative la présente invention.
Les quantités sont données en pourcentages massiques.

### Exemples 1 à 5: Influence des esters selon l'invention sur le SPF en présence de nano oxyde de titane.

Les inventeurs ont comparé l'influence des esters à groupement aromatique selon l'invention sur le SPF avec l'huile de ricin et le néopentanoate d'octyl-2 dodécyle, l'agent photoprotecteur étant un nano oxyde de titane.

On prépare les compositions N°1, 2, 3, 4 et 5 suivantes :

| | | Composition (%) | | | | |
|---|---|---|---|---|---|---|
| | Phase | Ex N°1 (Comparatif) | Ex N°2 (Comparatif) | Ex N°3 (Invention) | Ex N°4 (Invention) | Ex N°5 (Invention) |
| Huile de ricin | | 88 | | | | |
| Néopentanoate d'octyl-2 dodécyle | | | 88 | | | |
| Glyceryl dibenzoyl ricinoleate | A | | | 88 | | |
| Glyceryl tribenzoyl ricinoleate | | | | | 88 | |
| Glyceryl mono-dibenzoyl ricinoleate | | | | | | 88 |
| Nano oxyde de titane (UV-Titan M 262 commercialisé par la société Kemira) | B | 3 | 3 | 3 | 3 | 3 |
| Stéarate d'octa-cosanyle | C | 9 | 9 | 9 | 9 | 9 |
| | | 100 | 100 | 100 | 100 | 100 |

### Mode opératoire :

L'oxyde de titane (phase B ) est broyé dans une partie de la phase huileuse A.
La cire (phase C) est ajoutée ainsi que la partie restante de la phase A dans le broyat obtenu précedemment.
L'ensemble est homogéneisé à 100°C sous agitation avec un barreau aimanté.
Le mélange est coulé à 100°C dans un moule à 41-42°C.

### Mesure de SPF in vitro

On a déterminé pour chacun des exemples 1 à 5 le facteur de protection solaire (SPF) selon la méthode « in vitro » décrite par par B.L. Diffey dans J. Soc. Cosmet. Chem. 40, 127-133, (1989).
Les mesures ont été réalisées à l'aide d'un spectrophotomètre UV-visible SPF 290S de chez Optometrics muni d'une sphère d'intégration et d'une lampe Xénon, à température ambiante.

Chaque composition est appliquée sur un ruban adhésif TRANSPORE de chez 3M collé sur une lame de quartz, sous la forme d'un dépôt homogène et régulier à raison de 1,5 mg/cm².

### Résultats

| Exemples | | | | | |
|---|---|---|---|---|---|
| | N°1 | N°2 | N°3 | N°4 | N°5 |
| SPF | 4,18 | 2,54 | 4,32 | 5,40 | 5,64 |
| Ecart Type(+/-) | 0,30 | 0,27 | 0,22 | 0,68 | 0,64 |

Ces résultats montrent que les esters de l'invention permettent une augmentation significative du SPF des nano-oxydes de titane en comparaison avec les huiles connues telles que l'huile de ricin et le néopentanoate d'octyl-2 dodécyle.

### Exemples 6 à 9 : Influence des esters selon l'invention sur le SPF en présence de nano oxyde de zinc

Les inventeurs ont comparé l'influence des esters à groupement aromatique selon l'invention sur le SPF, avec l'huile de ricin et le néopentanoate d'octyl-2 dodécyle, l'agent photoprotecteur étant un nano oxyde de zinc.

On prépare les compositions N° 6, 7, 8 et 9 suivantes :

| | | Composition (%) | | | |
|---|---|---|---|---|---|
| | Phase | Ex N°6 (Comparatif) | Ex N°7 (Comparatif) | Ex N°8 (Invention) | Ex N°9 (Invention) |
| Huile de ricin | A | 83 | | | |
| Néopentanoate d'octyl-2 dodécyle | | | 83 | | |
| Glyceryl dibenzoyl ricinoleate | | | | 83 | |
| Glyceryl mono-dibenzoyl ricinoleate | | | | | 83 |
| Nano oxyde de titane (Z-Cote commercialisé par la société Sunsmart) | B | 5 | 5 | 5 | 5 |
| Stéarate d'octa-cosanyle | C | 12 | 12 | 12 | 12 |
| | | 100 | 100 | 100 | 100 |

On utilise le même mode opératoire que précédemment
Le SPF est mesuré selon la méthode de l'exemple précédent.

### Résultats

| Exemples | | | | |
|---|---|---|---|---|
| | N°6 | N°7 | N°8 | N°9 |
| SPF | 2,82 | 2,06 | 2,80 | 2,94 |
| Ecart Type(+/-) | 0,13 | 0,13 | 0,10 | 0,08 |

On constate que les esters de l'invention permettent une augmentation du SPF de compositions contenant un nano oxyde de zinc en comparaison avec le néopentanoate d'octyl-2 dodécyle et un SPF à peu près équivalent à celui obtenu avec l'huile de ricin.

### Exemples 10 à 13 : Influence des esters selon l'invention sur le SPF en présence d'un filtre organique liposoluble.

Les inventeurs ont comparé l'influence des esters à groupement aromatique selon l'invention sur le SPF avec le néopentanoate d'octyl-2 dodécyle, l'agent photoprotecteur étant l'octyl methoxycinnamate.

On prépare les compositions N°10, 11, 12 et 13 suivantes :

| | | Composition (%) | | | |
|---|---|---|---|---|---|
| | Phase | Ex N°10 (Comparatif) | Ex N°11 (Invention) | Ex N°12 (Invention) | Ex N°13 (Invention) |
| Néopentanoate d'octyl-2 dodécyle | | 85 | | | |
| Glyceryl monobenzoyl ricinoleate | A | | 85 | | |
| Glyceryl dibenzoyl ricinoleate | | | | 85 | |
| Glyceryl mono-dibenzoyl ricinoleate | | | | | 85 |
| Octyl méthoxycinnamate | B | 5 | 5 | 5 | 5 |
| Stéarate d'octa-cosanyle | C | 10 | 10 | 10 | 10 |
| | | 100 | 100 | 100 | 100 |

### Mode opératoire :

Les constituants des phases A et B sont mélangés et homogénéisés sous agitation, à température ambiante.
La cire (phase C) est ajoutée et l'ensemble est homogénéisé à 100°C sous agitation avec un barreau aimanté.
Le mélange est coulé à 100°C dans un moule à 40-42°C.

### Mesure de SPF in vitro

On a déterminé pour chacun des exemples 8 à 10 le facteur de protection solaire (SPF) selon la méthode « in vitro » décrite par B.L. Diffey dans J. Soc. Cosmet. Chem. 40, 127-133, (1989).
Les mesures ont été réalisées à l'aide d'un spectrophotomètre UV-visible SPF 290S de chez Optometrics muni d'une sphère d'intégration et d'une lampe Xénon.

Chaque composition est appliquée sur un ruban adhésif TRANSPORE de chez 3M collé sur une lame de quartz, sous la forme d'un dépôt homogène et régulier à raison de 1,5 mg/cm².

### Résultats

| Exemples | | | | |
|---|---|---|---|---|
| | N°10 | N°11 | N°12 | N°13 |
| SPF | 6,02 | 8,08 | 7,14 | 6,46 |
| Ecart Type(+/-) | 0,63 | 1,15 | 0,52 | 0,78 |

Ces résultats montrent que les esters de l'invention permettent une augmentation du SPF de filtres inorganiques tels que l'octyl méthoxycinnamate par rapport à une huile classique telle que l'octyl dodécyl néopentanoate.

### Exemple 14 : Base de soin pour les lévres

| | |
|---|---|
| -Glyceryl mono-dibenzoyl ricinoleate (FINSOLV BCO-115) | 22,58 % |
| -C12-15 Alkyl Benzoate | 20,78 % |
| -Phenyl Trimethicone | 5,19 % |
| -Di Isostearyl Malate | 7,27 % |
| -Tridecyl Trimellitate | 15,58 % |
| -Huile de Lanoline | 13,50 % |
| -BHT | 0,10% |
| -Cire de polyéthylène (MW=500) | 3,22 % |
| -Cire de candellila | 5,64 % |
| -Cire de Carnauba | 3,14 % |
| -Nano oxyde de titane (UV-TITAN M 262 de KEMIRA) | 3,00% |
| | 100,00 % |

### Mode opératoire:

On broie au broyeur tricylindre le nano oxyde de titane dans le glyceryl mono dibenzoyl ricinoleate.

On ajoute ensuite les autres constituants et l'on chauffe à 100 °C sous agitation magnétique jusqu'à fusion des cires et homogénéisation du mélange.
On coule alors le mélange à 100 °C dans les alvéoles d'un moule de manière a obtenir un stick.

### Exemple 15 : Rouge a lèvres

| | |
|---|---|
| -Glyceryl mono-dibenzoyl ricinoleate (FINSOLV BCO-115) | 15,58 % |
| -C12-15 Alkyl Benzoate | 20,78 % |
| -Phenyl Trimethicone | 5,19 % |
| -Di Isostearyl Malate | 7,27 % |
| -Tridecyl Trimellitate | 15,58 % |
| -Huile de Lanoline | 13,50 % |
| -BHT | 0,10 % |
| -Cire de polyéthylène (MW=500) | 3,22 % |
| -Cire de candellila | 5,64 % |
| -Cire de Carnauba | 3,14 % |
| -Nano oxyde de titane (UV-TITAN M 262 de KEMIRA) | 3,00 % |
| -FD et C Yellow 6 Al Lake | 3,37 % |
| -Oxyde de fer noir | 0,06 % |
| -DC Red 21 Al lake | 0,61 % |
| -DC Red 7 | 2,96 % |
| | 100,00% |

### Mode opératoire:

On broie au broyeur tricylindre le nano oxyde de titane dans le glyceryl mono-dibenzoyl ricinoleate.
On broie au broyeur tricylindre les pigments dans un mélange constitué du reste de la phase huileuse,de la lanoline et du BHT.
On rassemble les deux broyats, puis on ajoute les cires et l'on chauffe à 100 °C sous agitation magnétique jusqu'à fusion des cires et homogénéisation du mélange.
On coule alors la composition à 100 °C dans les alvéoles d'un moule de manière à obtenir un stick.

## Revendications

1. Composition cosmétique de soin ou de maquillage des matières kératiniques comprenant, dans un milieu physiologiquement acceptable, au moins un agent photoprotecteur capable de filtrer le rayonnement UV, une phase particulaire et au moins un ester à groupement aromatique, liquide à température ambiante, résultant de l'estérification par un acide aromatique d'au moins un groupe hydroxyle pendant ou en bout de chaîne d'un composé aliphatique hydroxylé choisi parmi les acides aliphatiques hydroxylés et leurs esters.

2. Composition cosmétique de soin ou de maquillage des matières kératiniques comprenant, dans un milieu physiologiquement acceptable, au moins un agent photoprotecteur capable de filtrer le rayonnement UV contenant un ou plusieurs filtres particulaires inorganiques et au moins un ester à groupement aromatique, liquide à température ambiante, résultant de l'estérification par un acide aromatique d'au moins un groupe hydroxyle pendant ou en bout de chaîne d'un composé aliphatique hydroxylé choisi parmi les acides aliphatiques hydroxylés et leurs esters.

3. Composition selon l'une des revendications précédentes, **caractérisée en ce que** l'agent photoprotecteur représente de 0,1 à 45% et de préférence de 0,5 à 25% du poids total de la composition.

4. Composition selon la revendication 2 ou 3, **caractérisée en ce que** les filtres particulaires inorganiques sont choisis parmi les pigments ou les nano pigments d'oxydes métalliques, traités ou non, capables de bloquer physiquement, par diffusion et/ou réflexion, le rayonnement UV.

5. Composition selon l'une des revendications 2 à 4, **caractérisée en ce que** les filtres particulaires inorganiques sont choisis parmi les nano pigments d'oxydes de titane, de zinc, de fer, de zirconium, de cérium, traités ou non, et leurs mélanges.

6. Composition selon la revendication 5, **caractérisée en ce que** les nano pigments sont choisis parmi les nano oxydes de titane ou de zinc.

7. Composition selon l'une des revendications 4 à 6, **caractérisée en ce que** les nano pigments contiennent des particules élémentaires dont la taille est comprise entre 5 et 500 nm, de préférence entre 10 et 250 nm, et mieux entre 10 et 100 nm.

8. Composition selon l'une des revendications 4 à 7, **caractérisée en ce que** les nano pigments représentent de 0,1 à 30 %, de préférence de 0,5 à 15% du poids total de la composition.

9. Composition selon l'une des revendications précédentes, **caractérisée en ce que** l'acide aromatique est choisi parmi l'acide benzoïque, l'acide phényl acétique, l'acide cinnamique, l'acide 3 phényl propanoïque, l'acide salicylique ; l'acide téréphtalique ; l'acide trimellitique ; l'acide pyromellitique et leurs mélanges.

10. Composition selon l'une des revendications précédentes, **caractérisée en ce que** l'acide aromatique est l'acide benzoïque.

11. Composition selon l'une des revendications précédentes, **caractérisée en ce que** les acides aliphatiques hydroxylés comprennent de 2 à 40 atomes de carbone, de préférence de 10 à 34 atomes de carbone et mieux de 12 à 28 atomes de carbone.

12. Composition selon l'une des revendications précédentes, **caractérisée en ce que** les acides aliphatiques hydroxylés comportent, en outre, de 1 à 20 groupes hydroxyle, de préférence de 1 à 10 groupes hydroxyle et mieux de 1 à 6 groupes hydroxyle, susceptible(s) d'être estérifié(s) par l'acide aromatique.

13. Composition selon l'une quelconque des revendications précédentes, **caractérisée en ce que** le composé aliphatique hydroxylé est choisi parmi :
i) les monoacides aliphatiques mono hydroxylés linéaires saturés de formule : 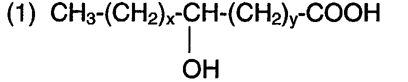 avec 0 ≤ x + y ≤ 37
ou
(2) HO-CH₂-(CH₂)ₓ-COOH
avec 0 ≤ x ≤ 38 ;
ii) les monoacides aliphatiques mono hydroxylés ramifiés saturés de formule : 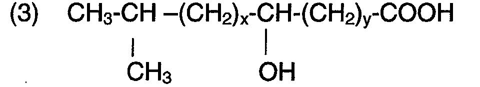 avec 0 ≤ x + y ≤ 35
ou (3') l'acide 2-éthyl 3-hydroxy caprylique de formule : 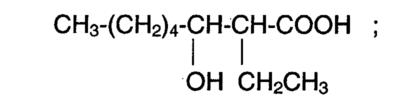
iii) les monoacides aliphatiques mono hydroxylés insaturés de formule : 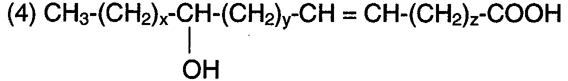 avec 0 ≤ x + y + z ≤ 35
ou 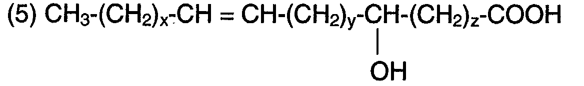 avec 0 ≤ x + y + z ≤ 35
ou
(6) HOCH₂-(CH₂)ₓ-CH = CH-(CH₂)_{y}-COOH
avec 0 ≤ x + y ≤ 36 ;
iv) les monoacides aliphatiques poly hydroxylés saturés de formule : 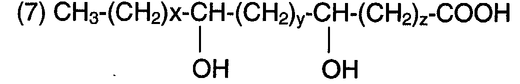 avec 0 ≤ x + y + z ≤ 36;
v) les polyacides aliphatiques mono hydroxylés saturés de formule : 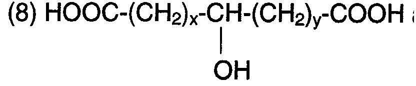 avec 0 ≤ x + y ≤ 37 ;
vi) les polyacides aliphatiques poly hydroxylés saturés ;
vii) les esters de monoacides aliphatiques mono hydroxylés linéaires saturés comme les esters de l'acide lactique et les esters de l'acide 12-hydroxy octadécanoïque ;
viii) les esters de monoacides aliphatiques mono hydroxylés insaturés tels que les esters de l'acide ricinoléique ;
ix) les esters de polyacides aliphatiques mono hydroxylés saturés ;
x) les esters de polyacides aliphatiques poly hydroxylés saturés ;
xi) les esters partiels ou totaux de polyol en C₂ à C₁₆ ayant réagit avec un acide aliphatique hydroxylé ;
et leurs mélanges.

14. Composition selon l'une quelconque des revendications précédentes, **caractérisée en ce que** le composé hydroxylé aliphatique est choisi parmi :
- l'acide lactique ; l'acide 12-hydroxy octadécanoïque ; l'acide α-hydroxy octadécanoïque ;
- l'acide glycolique ou l'acide junipérique ;
- l'acide leucinique ou l'acide 2-éthyl 3-hydroxy caprylique ;
- l'acide ricinoléique ;
- l'acide 3-hydroxy 4-hexanoïque ou l'acide oxynervonique ;
- l'acide 16-hydroxy 6-hexadécènoïque ;
- l'acide 9, 10-dihydroxy octadécanoïque, l'acide 9, 12-dihydroxy octadécanoïque, l'acide aleuritique, l'acide 9, 10, 12-trihydroxy octadécanoïque, l'acide hexahydroxy octa-décanoïque ou l'acide octahydroxy octadécanoïque ;
- l'acide malique ou l'acide citrique ;
- l'acide tartrique ;
- le lactate d'isostéaryle, le lactate issu d'alcool en C₁₂-C₁₃, le lactate d'octyl dodécyle, le lactate d'oléyle, le lactate de myristyle ;
- l'hydroxy stéarate d'éthyl 2-hexyle, l'hydroxy stéarate d'octyl docécyle, l'hydroxy stéarate d'isostéaryle, l'hydroxy stéarate d'isodécyle, le trihydroxy stéarate de glycéryle, l'hexahydroxy stéarate de dipentaérythrityle ;
- le ricinoléate de butyle, le ricinoléate d'octyl dodécyle, le ricinoléate de cétyle, le triricinoléate de glycéryle, l'huile de ricin ;
- le malate de diisostéaryle, le citrate de triisostéaryle, le citrate de trioctyl dodécyle ;
- le tartrate issu de dialcools en C₁₂-C₁₃ ramifiés ;
et leurs mélanges.

15. Composition selon l'une des revendications précédentes, **caractérisée en ce que** l'ester à groupement aromatique est un ester d'ester d'acide gras aliphatique dont le reste d'acide gras comporte au moins 12 atomes de carbone.

16. Composition selon l'une quelconque des revendications précédentes, **caractérisée en ce que** le composé hydroxylé aliphatique est choisi parmi les esters de l'acide ricinoléique, les esters de l'acide 12-hydroxy stéarique, les esters de l'acide lactique, les esters de l'acide hydroxy 14-eicosènoïque et leurs mélanges.

17. Composition selon l'une des revendications précédentes, **caractérisée en ce que** l'ester à groupement aromatique est choisi parmi le glyceryl monobenzoyl ricinoleate, le glyceryl mono-dibenzoyl ricinoleate, le glyceryl dibenzoyl ricinoleate, glyceryl tribenzoyl ricinoleate et leurs mélanges.

18. Composition selon l'une des revendications précédentes, **caractérisée en ce que** l'ester à groupement aromatique est le glyceryl mono-dibenzoyl ricinoleate.

19. Composition selon l'une des revendications précédentes, **caractérisée en ce que** l'ester à groupement aromatique est présent en une quantité allant de 0,1 à 99,9%, de préférence de 1 à 99 % et mieux de 5 à 90% du poids total de la composition.

20. Composition selon l'une des revendications précédentes, **caractérisée en ce que** le rapport ester/agent photoprotecteur va de 999 à 0,01 et mieux de 200 à 1.

21. Composition selon l'une des revendications précédentes, **caractérisée en ce qu'**elle contient des pigments et/ou des nacres.

22. Composition selon l'une des revendications précédentes, **caractérisée en ce qu'**elle contient au moins un corps gras additionnel choisi parmi les cires, les huiles, les corps gras pâteux à température ambiante, les gommes, les résines, les polymères lipophiles et leurs mélanges.

23. Composition selon l'une des revendications précédentes, **caractérisée en ce qu'**elle contient une cire.

24. Composition selon la revendication 22 ou 23, **caractérisée en ce que** le ou les corps gras additionnels représentent de 0,01 à 90% du poids total de la composition, de préférence, de 0,05 à 60% et mieux de 1 à 35%.

25. Composition selon l'une des revendications précédentes, **caractérisée en ce qu'**elle contient un ou plusieurs additifs choisis parmi les adoucissants, les antioxydants, les conservateurs, les opacifiants, les neutralisants, les stabilisants, les émollients, les silicones, les composés fluorés, les agents anti-mousse, les parfums, les tensioactifs, les polymères, les propulseurs, les agents alcalinisants ou acidifiants, les gélifiants lipophiles ou de corps gras liquides, les dispersants, les matières colorantes, les actifs cosmétiques ou dermatologiques et leur mélanges.

26. Composition selon la revendication précédente, **caractérisée en ce que** les additifs sont présents à raison de 0,001 à 30% du poids total de la composition et mieux de 0,005 à 10%.

27. Composition selon l'une des revendications précédentes, **caractérisée en ce qu'**elle se présente sous forme d'une solution huileuse, d'un gel huileux ou anhydre, d'une émulsion huile-dans-eau ou eau-dans-huile, d'une émulsion multiple, d'une dispersion d'huile dans de l'eau grâce à des vésicules.

28. Composition selon l'une des revendications précédentes, **caractérisée en ce qu'**il s'agit d'une composition protectrice de l'épiderme humain contre les rayons UV ou d'une composition solaire se présentant sous la forme d'un produit coulé, d'une pâte souple, d'une crème, d'un lait, d'une pommade, d'un gel, d'un gel crème, d'un stick ou d'un bâtonnet solide, d'une poudre, d'une mousse aérosol ou d'un spray.

29. Composition selon l'une des revendications précédentes **caractérisée en ce qu'**il s'agit d'une composition pour la protection des cheveux contre les rayons UV, se présentant sous forme de shampooing, d'après shampooing ou de lotion.

30. Composition selon l'une des revendications précédentes, **caractérisée en ce qu'**il s'agit d'une composition de maquillage des cils, des sourcils, de la peau et/ou des lèvres.

31. Composition selon l'une des revendications précédentes, **caractérisée en ce qu'**il s'agit d'une composition de maquillage des lèvres.

32. Composition selon l'une des revendications précédentes, **caractérisée en ce qu'**elle se présente sous forme anhydre.

33. Composition selon l'une des revendications précédentes, **caractérisée en ce qu'**elle se présente sous la forme solide à température ambiante.

34. Composition selon l'une des revendications précédentes, **caractérisée en ce qu'**elle est sous forme d'une composition destinée à la protection de la peau et/ou des lèvres et/ou des phanères contre les rayonnements UV, en particulier le rayonnement solaire.

35. Utilisation d'au moins un ester à groupement aromatique, liquide à température ambiante, résultant de l'estérification par un acide aromatique d'au moins un groupe hydroxyle pendant ou en bout de chaîne d'un composé aliphatique hydroxylé choisi parmi les acides aliphatiques hydroxylés et leurs esters, dans une composition cosmétique ou pour la fabrication d'une composition physiologique destinée à la protection de la peau et/ou des lèvres et/ou des phanères contre les rayonnements UV contenant au moins un agent photoprotecteur, pour améliorer le pouvoir photoprotecteur de ladite composition.

36. Utilisation d'au moins un ester à groupement aromatique, liquide à température ambiante, résultant de l'estérification par un acide aromatique d'au moins un groupe hydroxyle pendant ou en bout de chaîne d'un composé aliphatique hydroxylé choisi parmi les acides aliphatiques hydroxylés et leurs esters, dans une composition cosmétique solaire ou pour la fabrication d'une composition physiologique solaire contenant au moins un agent photoprotecteur.

37. Utilisation selon la revendication 35 ou 36, **caractérisée en ce que** l'ester à groupement aromatique est conforme à l'une quelconque des revendications 9 à 19.

38. Utilisation selon l'une des revendications 35 à 37, **caractérisée en ce que** l'agent photoprotecteur représente de 0,1 à 45% et de préférence de 0,5 à 25% du poids total de la composition.

39. Utilisation selon l'une des revendications 35 à 38, **caractérisée en ce que** l'agent photoprotecteur contient un ou plusieurs filtres organiques hydrophiles et/ou un ou plusieurs filtres organiques lipophiles et/ou un ou plusieurs filtres inorganiques actifs dans l'UV.

40. Utilisation selon la revendication 39, **caractérisée en ce que** les filtres organiques hydrophiles et/ou les filtres organiques lipophiles actifs dans l'UV sont choisis parmi les dérivés cinnamiques ; les dérivés de dibenzoylméthane ; les dérivés salicyliques, les dérivés du camphre ; les dérivés de triazine; les dérivés de la benzophénone ; les dérivés de β,β'-diphénylacrylate, les dérivés de benzimidazole ; les dérivés bis-benzoazolyle ; les dérivés de bis-hydroxyphénolbenzotriazole ; les dérivés de l'acide p-aminobenzoïque ; les polymères hydrocarbonés filtres, les silicones filtres et leurs mélanges.

41. Utilisation selon l'une des revendications 35 à 40, **caractérisée en ce que** l'agent photoprotecteur contient au moins un filtre particulaire inorganique choisi parmi les pigments ou les nano pigments d'oxydes métalliques, traités ou non, capables de bloquer physiquement, par diffusion et/ou réflexion, le rayonnement UV.

42. Utilisation selon la revendication 41, **caractérisée en ce que** les filtres particulaires inorganiques sont choisis parmi les nano pigments d'oxydes de titane, de zinc, de fer, de zirconium, de cérium, traités ou non, et leurs mélanges.

43. Utilisation selon la revendication 41 ou 42, **caractérisée en ce que** les nano pigments sont choisis parmi les nano oxydes de titane ou de zinc.

44. Utilisation selon l'une des revendications 41 à 43, **caractérisée en ce que** les nanopigments ont une taille comprise entre 5 et 500 nm, de préférence entre 10 et 250 nm, et mieux entre 10 et 100 nm.

45. Utilisation selon l'une des revendications 35 à 44, **caractérisée en ce que** le rapport ester/agent photoprotecteur va de 999 à 0,01 et mieux de 200 à 1.

46. Procédé cosmétique de protection de la peau, des lèvres et/ou des phanères d'êtres humains contre le rayonnement solaire, consistant à introduire, dans une composition cosmétique contenant au moins un agent photoprotecteur, une quantité efficace d'un ester à groupement aromatique, liquide à température ambiante, résultant de l'estérification par un acide aromatique d'au moins un groupe hydroxyle pendant ou en bout de chaîne d'un composé aliphatique hydroxylé choisi parmi les acides aliphatiques hydroxylés et leurs esters.

47. Procédé pour augmenter les propriétés de protection solaire d'une composition cosmétique contenant au moins un agent photoprotecteur, consistant à introduire dans ladite composition une quantité efficace d'un ester à groupement aromatique, liquide à température ambiante, résultant de l'estérification par un acide aromatique d'au moins un groupe hydroxyle pendant ou en bout de chaîne d'un composé aliphatique hydroxylé choisi parmi les acides aliphatiques hydroxylés et leurs esters.

48. Procédé pour augmenter le facteur de protection solaire d'une composition cosmétique contenant au moins un agent photoprotecteur, consistant à introduire dans ladite composition une quantité efficace d'un ester à groupement aromatique, liquide à température ambiante, résultant de l'estérification par un acide aromatique d'au moins un groupe hydroxyle pendant ou en bout de chaîne d'un composé aliphatique hydroxylé choisi parmi les acides aliphatiques hydroxylés et leurs esters.

49. Procédé selon l'une quelconque des revendications 46 à 48, **caractérisé en ce que** l'ester à groupement aromatique est conforme à l'une quelconque des revendications 9 à 19.
